(19) Europäisches Patentamt / European Patent Office / Office européen des brevets

(11) **EP 2 649 934 A1**

(12) **EUROPEAN PATENT APPLICATION**

(43) Date of publication:
**16.10.2013 Bulletin 2013/42**

(51) Int Cl.:
**A61B 5/00** *(2006.01)*

(21) Application number: **13161923.1**

(22) Date of filing: **02.04.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.04.2012 JP 2012090998**

(71) Applicant: **Canon Kabushiki Kaisha**
**Tokyo 146-8501 (JP)**

(72) Inventor: **Wanda, Koichiro**
**Tokyo, Tokyo 146-8501 (JP)**

(74) Representative: **TBK**
**Bavariaring 4-6**
**80336 München (DE)**

(54) **Object information acquiring apparatus and method for controlling same**

(57) Disclosed is a photoacoustic wave diagnosing apparatus including a holding unit that presses and holds an object during imaging; a photoacoustic measuring unit that measures information on the photoacoustic wave of the object pressed by the holding unit; an optical coefficient acquiring unit that acquires an optical coefficient based on the object in the pressed state; and a reconstruction unit that performs image reconstruction based on the information on a photoacoustic wave signal measured by the photoacoustic measuring unit and the optical coefficient acquired by the optical coefficient acquiring unit.

FIG. 1

**Description**

BACKGROUND OF THE INVENTION

Field of the Invention

The present invention relates to an object information acquiring apparatus and a method for controlling the same.

Description of the Related Art

Particularly in the medical field, studies have been actively conducted to develop imaging apparatuses that cause light irradiated from a light source such as a laser to be transmitted into an object to acquire information inside the object. As such an imaging apparatus, photoacoustic tomography (PAT) has been proposed.

PAT represents a technology in which light is applied to an object (living body in the medical field) and a photoacoustic wave generated when the light transmitted and diffused inside the object is absorbed into living tissues is received and analyzed to visualize information on optical properties inside the object (living body) (PHYSICAL REVIEW E 71, 016706 (2005): Non Patent Literature 1). Thus, biological information such as the distribution of optical property values, particularly, the distribution of light energy absorption density inside the object can be acquired.

Examples of information on optical properties acquired with the technology include the distribution of initial sound pressure or the distribution of the absorption density of light energy resulting from light irradiation. Such information can be used, for example, to identify the positions of malignant tumors accompanying the growth of new blood vessels. It is useful to generate and display three-dimensional reconstruction images based on information on optical properties in order to understand information inside living tissues, and such images are expected to be helpful for performing diagnoses in the medical field. As described in, for example, Japanese Patent Publication No. 4829934 (Patent Literature 1), there has also been proposed an apparatus that holds an object and acquires information on optical properties.

In PAT, the initial sound pressure $P_0$ of an acoustic wave generated from a light absorber inside an object can be expressed by the following formula (1).

$$P_0 = \Gamma \cdot \mu_a \cdot \Phi \qquad ...(1)$$

Here, $\Gamma$ represents a Gruneisen coefficient, which is obtained by dividing the product of a volume expansion coefficient $\beta$ and the square of a sound velocity c by a specific heat at constant pressure CP. It is known that $\Gamma$ has an almost constant value when the object is determined. $\mu_a$ represents the light absorption coefficient of the light absorber. $\Phi$ represents a light amount (light amount applied to the absorber and also called light fluence) at a local region.

In PAT, the sound pressure P representing the size of the acoustic wave transmitted inside the object is measured, and the distribution of initial sound pressure is calculated from the measurement result of sound pressure at each time. Each value of the calculated distribution of the initial sound pressure is divided by the Gruneisen coefficient $\Gamma$, whereby the distribution of the product of $\mu_a$ and $\Phi$, i.e., the distribution of the absorption density of the light energy of the object can be acquired.

As shown in the formula (1), it is necessary to calculate the distribution of the light amount $\Phi$ inside the object in order to acquire the distribution of the light absorption coefficient $\mu_a$ from the distribution of the initial sound pressure $P_0$. Assuming that uniform light is transmitted inside the object like a plane wave when a region substantially larger in size than the thickness of the object is irradiated with the light, the distribution of the light amount $\Phi$ inside the object can be expressed by the following formula (2).

$$\Phi = \Phi_0 \cdot e \times p(-\mu_{eff} \cdot d) \qquad ...(2)$$

Here, $\mu_{eff}$ represents the average equivalent attenuation coefficient of the object. $\Phi_0$ represents the amount of the light incident on the object from a light source (the amount of the light at the front surface of the object). Further, d represents the distance between a region (light irradiating region) at the front surface of the object irradiated with the light from the light source and the light absorber inside the object. According to technology described in Japanese Patent Publication No. 4829934, a living body is irradiated with uniform light under several conditions to calculate the average equivalent attenuation coefficient $\mu_{eff}$ of the object. Then, the distribution of the light amount $\Phi$ is calculated based on the formula (2), and the distribution of the light absorption coefficient $\mu_a$ inside the object can be acquired based on the formula (1) using the distribution of the light amount Φ.

Further, Japanese Patent Application Laid- open No. 2011- 217914 (Patent Literature 2) discloses a method for performing imaging with PAT in which the transmission of light inside an object depends on two or more illumination conditions and for estimating the distribution of a light absorption coefficient inside the object.

In performing imaging with PAT in the related art, it is required to consider the attenuation amount of light inside an object in order to use the optical coefficient of the object as represented by the absorption coefficient of the light. A three-dimensional image based on a photoacoustic wave signal is reconstructed in consideration of the attenuation amount of light at each position of an object. To this end, image reconstruction using the accurate optical coefficient of the object is required to improve image quality in imaging and the performance of analysis processing in PAT.

Patent Literature 1: Japanese Patent Publication No. 4829934
Patent Literature 2: Japanese Patent Application Laid-open No. 2011-217914

Non Patent Literature 1: PHYSICAL REVIEW E 71, 016706 (2005)

SUMMARY OF THE INVENTION

In PAT, the photoacoustic wave of an object is measured, and the attenuation amount of light is calculated using the average of the non-uniform optical coefficients of a living body for each unit region as background optical coefficient. Then, correction is made at each position based on the attenuation amount of the light to perform image reconstruction using the distribution of the final absorption coefficients of the light. In the living body, however, body fluids and tissue forms themselves are likely to change unlike in the measurement of objects constituted of single substances. Therefore, the average of the optical coefficients for each unit region may also change for each calculation of the photoacoustic wave.

As a method for calculating the average of optical coefficients to be applied to image reconstruction (background optical coefficient), there has been known one using a value measured by another optical measuring apparatus at time other than imaging with PAT and a standard value according to age or the like, besides the method described in Japanese Patent Publication No. 4829934. However, in any method, it is inevitable that the average of optical coefficients for each unit region of a living body deviates from an optimum value at the measurement of a photoacoustic wave due to a change in the state of the living body. As a result, the accuracy of image reconstruction may be reduced.

Further, in the apparatus described in Japanese Patent Publication No. 4829934, an object is pressure- held so as to be fixed. In this case, the state of the object is changed by pressing, and thus it is difficult to reproduce the same state as that of a previous object at the next pressure holding. An optical coefficient for each unit region of the object also changes for each pressure holding. Therefore, even if the optical coefficient is measured using a measuring apparatus at time other than imaging with PAT, it cannot be said that the optical coefficient is an appropriate value representing the state of the object at the measurement of a photoacoustic wave. In other words, even if the optical coefficient is calculated, the state of the object changes at the application of the optical coefficient. For this reason, the accuracy of image reconstruction cannot be improved if the optical coefficient of the object in the same pressure- holding state as the time of the measurement of the photoacoustic wave is not applied.

In view of the above problems, the present invention is developed to acquire an accurate value based on the state of an object during imaging as the optical coefficient of the object for use in image reconstruction with PAT.

The present invention in its first aspect provides an object information acquiring apparatus as specified in claims 1 to 5.

The present invention in its second aspect provides a method for controlling an object information acquiring apparatus as specified in claim 6.

According to the present invention, it is possible to acquire an accurate value based on the state of an object during imaging as the optical coefficient of the object for use in image reconstruction with PAT.

Further features of the present invention will become apparent from the following description of exemplary embodiments with reference to the attached drawings.

BRIEF DESCRIPTION OF THE DRAWINGS

FIG. 1 is a diagram showing the configuration of a photoacoustic wave diagnosing apparatus according to a first embodiment;
FIG. 2 is a diagram showing the configuration of an information processing part according to the first embodiment;

FIG. 3 is a diagram showing the configuration of a photoacoustic wave signal measuring part according to the first embodiment;
FIG. 4 is a flowchart showing the outline of the processing procedure of the photoacoustic wave diagnosing apparatus;
FIG. 5 is a flowchart showing the processing procedure of the information processing part;
FIG. 6 is a flowchart showing the processing procedure of the photoacoustic wave signal measuring part;
FIG. 7 is a diagram showing the configuration of the photoacoustic wave diagnosing apparatus according to a second embodiment; and
FIG. 8 is a diagram showing the configuration of the information processing part according to the second embodiment.

DESCRIPTION OF THE EMBODIMENTS

Hereinafter, a description will be given of the preferred embodiments of the present invention with reference to the drawings. Note, however, that the sizes, materials, shapes of constituents described below and the relative arrangements between the constituents may be appropriately changed depending on the configurations and various conditions of an apparatus to which the emb odiments of the present invention are applied, and are not intended to limit the scope of the embodiments of the present invention to the following information.

A photoacoustic wave diagnosing apparatus according to the embodiments of the present invention includes an apparatus based on a photoacoustic effect in which an acoustic wave generated inside an object when the object is irradiated with light (electromagnetic wave) is received to acquire property information inside the object as image data. Herein, measuring a photoacoustic wave and making an image of the inside of the object like this will be called the imaging of the object. Light irradiation according to the embodiments of the present invention includes, besides irradiation for imaging the object, light irradiation for estimating the optical coefficient of the object.

Property information inside the object includes the distribution of the source of the acoustic wave generated by the light irradiation, the distribution of initial sound pressure inside the object, the distribution of the absorption density of light energy and the distribution of absorption coefficients derived from the distribution of initial sound pressure, and the distribution of the concentration of substances constituting tissues. Examples of the distribution of the concentration of substances include the distribution of the saturation degrees of oxygen and the distribution of the concentration of oxidized/reduced hemoglobin. Since such property information is also called object information, the photoacoustic wave diagnosing apparatus according to the embodiments of the present invention can also be called an object information acquiring apparatus.

The acoustic wave according to the embodiments of the present invention is typically an ultrasonic wave and includes an elasticity wave called a sound wave, an ultrasonic wave, or an acoustic wave. The acoustic wave generated by the photoacoustic effect is called a photoacoustic wave or a photoultrasonic wave. An acoustic detector (e.g., probe) receives the acoustic wave generated or reflected inside the object.

<First Embodiment>

The photoacoustic wave diagnosing apparatus according to a first embodiment measures both a photoacoustic wave signal for imaging and a photoacoustic wave signal for estimating an optical coefficient, while confirming a change in the holding state of the object. Then, the photoacoustic wave diagnosing apparatus estimates the optical coefficient of the object for imaging based on the photoacoustic wave signal for estimating the optical coefficient and applies the same to image reconstruction.

(Configuration of Apparatus)

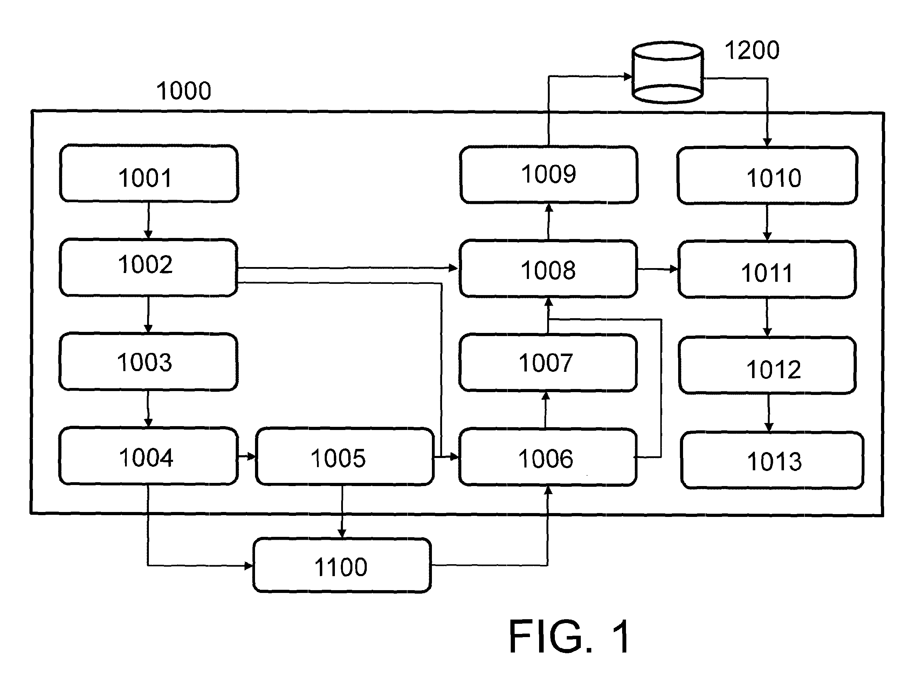

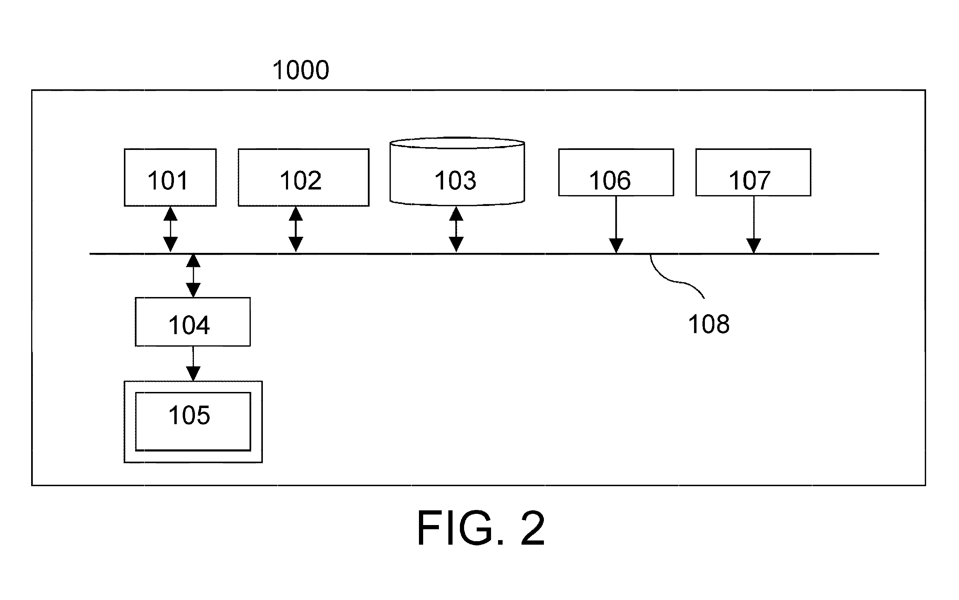

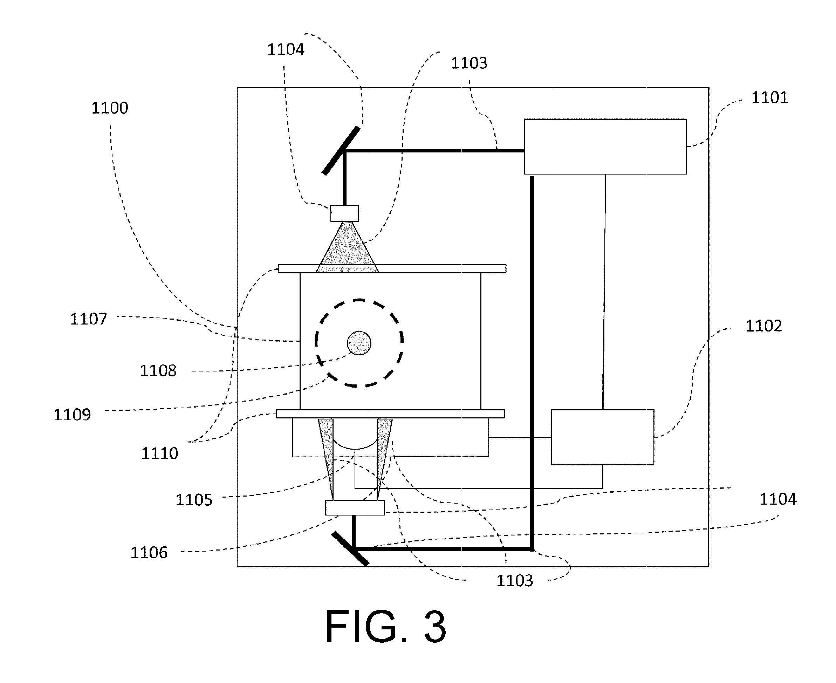

FIG. 1 is a block diagram showing the schematic functional configuration of the photoacoustic wave diagnosing apparatus according to the embodiment. The photoacoustic wave diagnosing apparatus according to the embodiment is composed of an information processing part 1000 and a photoacoustic wave signal measuring part 1100. FIG. 2 shows an example of an equipment configuration for implementing the information processing part 1000 of the photoacoustic wave diagnosing apparatus according to the embodiment. FIG. 3 shows an example of an equipment configuration for implementing the photoacoustic wave signal measuring part 1100.

(Photoacoustic Wave Signal Information)

The photoacoustic wave signal measuring part 1100 controls the measurement of the photoacoustic wave signal based on a photoacoustic wave measuring method instructed by the information processing part 1000. Then, the photoacoustic wave signal measuring part 1100 generates photoacoustic wave signal information based on an acoustic wave detected by each of the elements of an acoustic wave detector 1105 and transmits the same to the information processing part 1000.

Here, the acoustic wave detector 1105 shown in FIG. 3 is a probe that detects the acoustic wave with the elements arranged on the receiving surface thereof and converts the same into an electric signal (photoacoustic wave signal) . The acoustic wave detector 1105 detects a photoacoustic wave 1109 generated when an optical unit 1104 irradiates an object 1107 with light. The photoacoustic wave signal information described above includes a photoacoustic wave signal itself and information on the photoacoustic wave signal. The information on the photoacoustic wave signal includes information on, for example, the positions, sensitivity, directivity, or the like of the elements of the acoustic wave detector 1105. In addition, the information on the photoacoustic wave signal includes information on conditions for acquiring the photoacoustic wave signal such as imaging parameters for acquiring the acoustic wave. Such information is required to perform image reconstruction using the photoacoustic wave signal.

Further, out of the photoacoustic wave signal information, the information on the photoacoustic wave signal may include various information according to a photoacoustic wave measuring method. According to the embodiment, the information on the photoacoustic wave signal includes information on the photoacoustic wave signal for estimating the optical coefficient. In addition, according to a second embodiment, the information on the photoacoustic wave signal includes the optical coefficient acquired by a measuring apparatus. Moreover, the photoacoustic wave signal information may also include information on the control of the light source of irradiation light for generating the acoustic wave and information on holding and pressing of the object.

When the photoacoustic wave signal measuring part 1100 moves the probe to detect the acoustic wave, a scanning region in which the acoustic wave is detected by the probe is regarded as a receiving region and the position of the element that detects the acoustic wave is regarded as an element position at the receiving region. In this case, the photoacoustic wave signal measuring part 1100 generates the photoacoustic wave signal information on the position of the receiving region in a coordinate system inside the apparatus and on the element position at the receiving region.

Out of the photoacoustic wave signal information, the photoacoustic wave signal itself may be stored after being measured or may be stored after being subjected to correction such as element sensitivity correction and gain correction. In addition, it may be possible to repeatedly perform the irradiation of light and the reception of the acoustic wave several times at the same position on the object and average and store acquired photoacoustic wave signals. Note that even if the irradiation of the light and the reception of the acoustic wave are performed at the same position on the object, the detection may not be necessarily performed by the same element of the probe. If an element having the same ability detects the signal at the same position on the receiving region during the movement of the probe, the signal can be regarded as a signal at the same position.

Out of the information to be used for image reconstruction, information causing no problem as a static constant is stored in a main memory 102 and a magnetic disk 103 of the information processing part 1000 in advance. On the other hand, information dynamically set for each imaging is transmitted from the photoacoustic wave signal measuring part 1100 to the information processing part 1000 as part of the photoacoustic wave signal information.

According to the embodiment, the photoacoustic wave signal for estimating the optical coefficient is measured by the same light irradiation method as in the case of the photoacoustic wave signal for imaging. However, in order to acquire the photoacoustic wave signal for estimation, the photoacoustic wave signal may be required to be measured by a light irradiation method different from that in the case of imaging.

An example of the control of the light irradiation method includes the control of the direction of the irradiation light with respect to the object. That is, the direction of the irradiation light is selected from among the three directions of, for example, a forward direction, a backward direction, and a two-way direction. The forward direction is a direction in which the receiving surface of the photoacoustic wave detector 1105 is irradiated with the light from the front side thereof. The backward direction is a direction in which the receiving surface of the photoacoustic wave detector 1105 is irradiated with the light from the back side thereof. The two-way direction is a direction in which the receiving surface of the photoacoustic detector 1105 is irradiated with the light from both the front and back sides thereof. Even if the object in the same state is irradiated with the light, the transmission of the light inside the object varies depending on from which of the three directions the light is irradiated. As will be described in detail below, the direction and transmission of the light are needed to be considered for estimating the optical coefficient.

(Information Processing Part)

Next, a description will be given of the constituents of the information processing part 1000.

The information processing part 1000 acquires instructions on imaging from the user. Then, the information processing part 1000 determines a photoacoustic wave measuring method considering the image quality of a reconstruction image at a region to be imaged, and notifies the photoacoustic wave signal measuring part 1100 of the method. In addition, the information processing part 1000 performs three-dimensional image reconstruction using photoacoustic wave signal information acquired from the photoacoustic wave signal measuring part 1100 to display imaging data.

The information processing part 1000 has an imaging instruction information acquiring unit 1001, an optical coefficient measuring method determining unit 1002, a photoacoustic wave measuring method determining unit 1003, and a photoacoustic wave measuring method instructing unit 1004. In addition, the information processing part 1000 has an object state monitoring unit 1005, a photoacoustic wave signal information acquiring unit 1006, an optical coefficient estimating unit 1007, and a reconstruction processing unit 1008. Moreover, the information processing part 1000 has a data recording unit 1009, a data acquiring unit 1010, a data analyzing unit 1011, a display information generating unit 1012, and a displaying unit 1013.

The imaging instruction information acquiring unit 1001 acquires instructions on imaging (imaging instruction information) input by the user via an inputting unit 106. Examples of the imaging instruction information include an imaging region and settings on imaging functions using various parameters. In addition, examples of the imaging instruction information include information as to whether the optical coefficient is to be estimated during imaging and information as to whether measurement for estimating the optical coefficient is to be performed. The case in which the imaging instruction information acquiring unit 1001 is instructed by the user to measure the optical coefficient rather than estimating the same will be described in the second embodiment. The imaging instruction information acquiring unit 1001 transmits the input imaging instruction information to the optical coefficient measuring method determining unit 1002.

The imaging region is a three-dimensional region inside an object to be subjected to imaging. In the following description, the imaging region will basically refer to a region in which the photoacoustic wave signal for estimating the optical coefficient is measured. For example, the photoacoustic wave signal for estimating the optical coefficient is basically acquired in such a manner that the photoacoustic wave generated from the whole or some region of the object inside the imaging region is detected.

However, the region in which the photoacoustic wave signal for estimating the optical coefficient is measured is not necessarily limited to a region inside the imaging region. For example, if there is a case in which a region inside the imaging region is not suitable for estimating and measuring the optical coefficient but a region outside the imaging region is suitable for estimating and measuring the optical coefficient, it is also possible to specify any region outside the imaging region.

The optical coefficient measuring method determining unit 1002 determines whether the optical coefficient to be applied to image reconstruction is estimated and determines a measuring method based on the imaging instruction information to generate optical coefficient measuring information. The optical coefficient measuring method determining unit 1002 transmits the optical coefficient measuring information to the photoacoustic wave measuring method determining unit 1003, the photoacoustic wave signal information acquiring unit 1006, and the reconstruction processing unit 1008 together with the imaging instruction information.

The photoacoustic wave measuring method determining unit 1003 determines a specific photoacoustic wave measuring method based on the imaging instruction information and the optical coefficient measuring information. The photoacoustic wave measuring method determining unit 1003 generates photoacoustic wave measuring information in which instruction information items required to measure the photoacoustic wave signal for imaging or the photoacoustic wave signal for estimating the optical coefficient are put together, and transmits the same to the photoacoustic wave measuring method instructing unit 1004.

The photoacoustic wave measuring method instructing unit 1004 instructs the photoacoustic wave signal measuring part 1100 to start or stop measuring the photoacoustic wave signal. Further, during imaging, the photoacoustic wave measuring method instructing unit 1004 inquires the object state monitoring unit 1005 about the state of the object for confirmation.

During the measurement of the photoacoustic wave, the object state monitoring unit 1005 monitors whether there is no change in the holding state of the object. The object state monitoring unit 1005 notifies the photoacoustic wave signal information acquiring unit 1006 of the fact that there is no change in the state of the object, while acquiring the photoacoustic wave signal information during imaging accompanying the estimation of the optical coefficient. The object state monitoring unit 1005 may perform the notification at any timing, but it periodically performs the notification from the start to the end of the measurement of the photoacoustic wave according to the embodiment. Further, when determining that there is a change in the state of the object, the object state monitoring unit 1005 notifies the photoacoustic wave measuring method instructing unit 1004 of the fact that there is a change in the state of the object to stop the photoacoustic wave measuring processing.

The photoacoustic wave signal information acquiring unit 1006 receives the photoacoustic wave signal information transmitted from the photoacoustic wave signal measuring part 1100. Then, the photoacoustic wave signal information acquiring unit 1006 transmits the photoacoustic wave signal information for estimating the optical coefficient to the optical coefficient estimating unit 1007 and the photoacoustic wave signal information for imaging to the reconstruction processing unit 1008.

The optical coefficient estimating unit 1007 estimates the optical coefficient of the object based on the photoacoustic wave signal for estimating the optical coefficient. The optical coefficient estimating unit 1007 transmits the estimated value of the optical coefficient to the reconstruction processing unit 1008.

The reconstruction processing unit 1008 performs image reconstruction for each point at the imaging region using the photoacoustic wave signal information to generate a three-dimensional reconstruction image (volume data). In performing image reconstruction, the reconstruction processing unit 1008 uses the value of the optical coefficient estimated by the optical coefficient estimating unit 1007 and the photoacoustic wave signal information transmitted from the photoacoustic wave signal information acquiring unit 1006. Note here that the reconstruction processing unit 1008 may perform correction on the reconstruction image, such as correction for a case in which the intensity of light is not uniform inside a reconstruction region.

In addition, the reconstruction processing unit 1008 calculates the distribution of initial sound pressure and the distribution of light absorption coefficients inside the object. At this time, the light absorption coefficient is calculated by using value of the estimated optical coefficient as the background optical coefficient. Since the degree of light absorption inside the object varies depending on the wavelength of irradiation light inside the object, the reconstruction processing unit 1008 can make an image of a difference in composition inside the object. For example, using the irradiation light of a wavelength strongly absorbed by reduced hemoglobin and the irradiation light of a wavelength strongly absorbed by oxidized hemoglobin, the reconstruction processing unit 1008 can calculate the degree of the saturation of oxygen and make an image of the distribution of the degree of the saturation of oxygen. The reconstruction processing unit 1008 makes an image of such property information or combines the information with the result of other analysis processing according to the purpose of diagnosis, thereby making it possible to generate image data in various forms.

Further, the reconstruction processing unit 1008 transmits the generated reconstruction image, the imaging instruction information, the photoacoustic wave signal information, and the estimated value of the optical coefficient to the data recording unit 1009. However, when immediately displaying the reconstruction image regardless of whether data is recorded, the reconstruction processing unit 1008 also transmits them to the data analyzing unit 1011.

The data recording unit 1009 generates recording data based on the reconstruction image, the information on reconstruction, the imaging instruction information, the photoacoustic wave signal information, the estimated value of the optical coefficient, and the like transmitted from the reconstruction processing unit 1008.

The generated recording data is in the form of volume data in which voxel space corresponding to the imaging region is divided at a pitch specified by image reconstruction. The volume data may have data containing prescribed information. The data may be configured in any data format. As an example, the format of digital imaging and communications in medicine (DICOM), which is a standard format for medical images, can be used. There is no particular information on the photoacoustic wave diagnosing apparatus as a standard format. However, by storing information unique to the photoacoustic wave diagnosing apparatus in a private tag, the data recording unit 1009 can record information on the measurement of the photoacoustic wave while maintaining the versatility of DICOM.

The data recording unit 1009 stores the generated data in a storage medium like the magnetic disk 103 as a recording data file 1200. An actual storage medium is not limited to a magnetic disk, and the data recording unit 1009 may store the generated data in other information processing apparatuses or storage media via a network.

The data acquiring unit 1010 acquires the recording data stored in the recording data file 1200 by using a communicating unit responding to the storage medium. The data acquiring unit 1010 transmits the acquired recording data to the data analyzing unit 1011.

The data analyzing unit 1011 analyzes the recording data received from the data acquiring unit 1010 to extract the reconstruction image generated by the reconstruction processing unit 1008 and the information on the irradiation of light acquired by the photoacoustic wave signal information acquiring unit 1006 from the photoacoustic wave signal measuring part 1100. Then, the data analyzing unit 1011 prepares management information put together for each imaging data. When directly receiving the reconstruction image and the relevant information from the reconstruction processing unit 1008, the data analyzing unit 1011 also prepares the management information for each imaging data. The data analyzing unit 1011 transmits the management information on the imaging data including the reconstruction image to the display information generating unit 1012.

The display information generating unit 1012 generates display information on the reconstruction image and display information on a region having quantitativeness.

As for the display of the reconstruction image, the reconstruction image is used as the display information without being subjected to any special conversion if it is a plane image and falls within a range at which the reconstruction image can be displayed as it is at the brightness value of the display. If the reconstruction image is a three-dimensional image such as volume data, the display information generating unit 1012 generates the display information in any method such as volume rendering, multi planar reconstruction (MPR), and maximum intensity projection (MIP). In addition, if the range of the voxel value exceeds the range of the brightness value of the display, the display information generating unit 1012 generates the display information so as to fall within the range of a pixel value at which the display information can be displayed on the display. The display information includes information capable of displaying at least the reconstruction image.

As an example of the display information based on information having quantitativeness, the display information generating unit 1012 allocates a boundary line that allows the identification of a region having quantitativeness or a display color that is different for each region and shows the presence or absence of quantitativeness. Moreover, the display information generating unit 1012 can also generate the display information having an annotation such as text information that shows the signal value of a region having quantitativeness and the properties and the analysis result of the region, or the like.

The displaying unit 1013 is a displaying device such as a graphic card, a liquid crystal, and a cathode ray tube (CRT) display that displays the generated display information and displays thereon the display information transmitted from the display information generating unit 1012.

Note that in the description of the photoacoustic wave diagnosing apparatus according to the embodiment of the present invention, the photoacoustic wave signal measuring part 1100 and the information processing part 1000 will be individually described. A specific example of the photoacoustic wave diagnosing apparatus includes a combination of a measuring apparatus such as a digital mammography and a controlling apparatus such as a personal computer (PC). Alternatively, a single image information acquiring apparatus including the photoacoustic wave signal measuring part 1100 and the information processing part 1000 may be used as the photoacoustic wave diagnosing apparatus. For example, the photoacoustic wave diagnosing apparatus may also be implemented by an apparatus configuration such as a modality in which a general ultrasonic wave diagnosing apparatus has functions corresponding to the photoacoustic wave signal measuring part 1100 and the information processing part 1000 according to the embodiment of the present invention.

FIG. 2 is a diagram showing the basic configuration of a computer that implements the functions of each unit of the information processing part 1000 with software.

A central processing unit (CPU) 101 mainly controls the operations of each constituent of the information processing part 1000. The main memory 102 stores therein a control program executed by the CPU 101 and provides a work area for the execution of the program by the CPU 101. The magnetic disk 103 stores therein an operating system (OS), the device drivers of peripheral devices, various application software including a program for performing the processing of a flowchart that will be described below, or the like. A displaying memory 104 temporarily stores therein display data for the monitor 105.

The monitor 105 is, for example, a CRT display or a liquid crystal monitor and displays thereon an image based on data transmitted from the displaying memory 104. The inputting unit 106 is, for example, a mouse or a keyboard that allows an operator to perform pointing input, character input, or the like. According to the embodiment of the present invention, the operator performs operations and inputs instruction information via the inputting unit 106.

An I/F 107 is used to exchange various data between the information processing part 1000 and the outside, and constituted of IEEE1394, US5, an Ethernet port (TM), or the like. Data acquired via the I/F 107 is imported into the main memory 102. The functions of the photoacoustic wave signal measuring part 1100 are implemented via the I/F 107. Note that the constituents described above are connected so as to communicate with each other via a common bus 108.

(Photoacoustic Wave Signal Measuring Part)

FIG. 3 is a diagram showing an example of the configuration of the photoacoustic wave signal measuring part 1100 of the photoacoustic wave diagnosing apparatus according to the embodiment of the present invention.

A light source 1101 is the light source of irradiation light such as a laser and a light emitting diode for irradiating the object. As the irradiation light, the light of a wavelength expected to be strongly absorbed by a specific one of components constituting the object is used.

A controlling unit 1102 controls the light source 1101, the optical unit 1104, the acoustic wave detector 1105, and a position controlling unit 1106. In addition, the controlling unit 1102 amplifies the electric signal of the acoustic wave acquired by the acoustic wave detector 1105 to be converted from an analog signal to a digital signal. Further, the controlling unit 1102 may perform various signal processing and various correction processing. Furthermore, the controlling unit 1102 transmits the photoacoustic wave signal from the photoacoustic wave signal measuring part 1100 to external equipment such as the information processing part 1000 via an interface (not shown).

As for the control of the laser, the controlling unit 1102 controls the timing, waveform, strength, or the like of laser irradiation. As for the control of the position controlling unit 1106 of the acoustic wave detector 1105, the controlling unit 1102 moves the acoustic wave detector 1105 to an appropriate position. Further, the controlling unit 1102 performs various control to measure the photoacoustic wave signal detected by the acoustic wave detector 1105 in synchronization with the timing of the laser irradiation. Moreover, the controlling unit 1102 performs signal processing in which the photoacoustic wave signals for each element acquired by several laser irradiation operations are added and averaged to calculate the average of the photoacoustic wave signals for each element.

The optical unit 1104 is an optical component such as a mirror that reflects light and a lens that condenses and enlarges light and changes the shape of light. As such, an optical component that causes the object 1107 to be irradiated with light 1103 emitted from the light source 1101 in a desired form is used. Alternatively, with the arrangement of a plurality of the light sources 1101 or a plurality of the optical units 1104, it is also possible to irradiate the object 1107 with the light 1103 from various directions. The light 1103 irradiated from the light source 1101 may be transmitted to the object 1107 via optical waveguides such as optical fibers.

When the object 1107 is irradiated with the light 1103 generated from the light source 1101 via the optical unit 1104 by the control of the controlling unit 1102 under such a configuration, a light absorber 1108 inside the object 1107 absorbs the light 1103 and radiates the photoacoustic wave 1109. In this case, the light absorber 1108 corresponds to a sound source. If the object 1107 is held by a pair of holding plates (flat plates), the object 1107 may be irradiated with the light 1103 from the side of one of the flat plates or may be irradiated with the light 1103 from the sides of both flat plates.

The acoustic wave detector 1105 is composed of a transducer based on a piezoelectric phenomenon, a transducer based on light resonance, a transducer based on a change in capacity, or the like. However, any acoustic wave detector may be used so long as the acoustic wave can be detected. The acoustic wave detector 1105 may detect the acoustic wave in a state of directly contacting the object 1107 or may detect the acoustic wave over the flat plates 1110 that press the object 1107.

In the acoustic wave detector 1105 according to the embodiment, a plurality of elements (detecting elements) is two-dimensionally arranged. With such two-dimensionally arranged elements, it is possible to simultaneously detect the acoustic wave at a plurality of places, reduce detection time, and reduce the influence of the vibrations or the like of the object 1107. In addition, an acoustic impedance matching agent such as gel and water (not shown) may be applied between the acoustic wave detector 1105 and the object 1107 to reduce the reflection of the acoustic wave.

If a region for irradiating the object 1107 with the light 1103 and the acoustic wave detector 1105 are movable, it is possible to acquire the photoacoustic wave at a wider region. To this end, the optical unit 1104 is configured to be movable, or a movable mirror or the like is used. Upon receiving instructions from the controlling unit 1102, the position controlling unit 1106 moves the region and the acoustic wave detector 1105 by, for example, a motor. At this time, the position controlling unit 1106 performs control such that the region for irradiating the object 1107 with the light 1103 and the receiving region of the acoustic wave detector 1105 are caused to move in synchronization with each other.

The acoustic wave detector 1105 can move in various ways. For example, if the surface of the acoustic wave detector 1105 having the element of the probe arranged thereon is rectangle, the probe is caused to move by a distance corresponding to the vertical or horizontal length thereof and stop at the corresponding positions to detect the acoustic wave. Thus, the probe can be regarded as one in which the elements corresponding to moving times are arranged at the same element pitches. Alternatively, the probe may be caused to sequentially reciprocate to receive the acoustic wave. If the probe is caused to shift little by little at the reciprocal movements, it can measure the acoustic wave at a wider region.

Moreover, the controlling unit 1102 also generates information required to extract information on a region having quantitativeness out of an imaging region. The information includes an imaging position, the imaging region, the amount of the light irradiated with respect to the object 1107 during imaging, or the like.

The photoacoustic wave signal measuring part 1100 acquires the photoacoustic wave signal required to make an image of the imaging region specified by the user. The imaging region is a three-dimensional region specified for each objective imaging. The imaging region may be specified by any method. For example, the coordinates of each apex of a cuboid or a mathematical formula may be input to specify the imaging region. Further, the user may specify a rectangular region on a camera image capturing the object 1107 by a mouse and specify the imaging region based on a plane in which the region is projected onto the object 1107 and information on the depth direction. In this case, the camera image is taken over a transparent flat plate to measure the thickness of the object 1107 from the flat plate, thereby making it possible to specify a cuboid as the imaging region. Note that the imaging region is not necessarily a cuboid.

(Outline of Processing at Photographing)

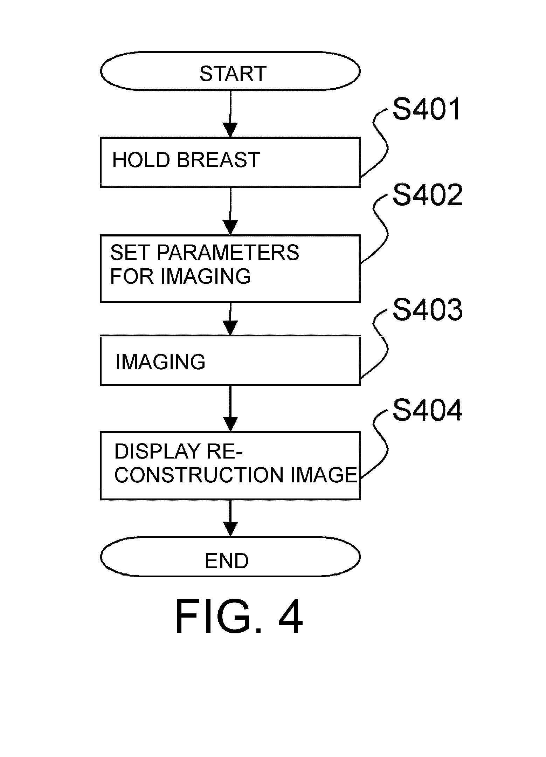

Next, a description will be given of a specific processing procedure according to the embodiment using flowcharts shown in FIGS. 4 to 6. FIG. 4 is a flowchart showing the outline of an imaging procedure when a doctor or a laboratory technician images the breast of the object by the photoacoustic wave diagnosing apparatus according to the embodiment of the present invention. The flow of the flowchart shows a general procedure, and processing unique to the embodiment of the present invention is included in each step as will be described later. The flow starts from a state in which the breast of the object is placed at the holding position of the photoacoustic wave diagnosing apparatus.

In step S401, the operator controls the position of the flat plates 1110 via the inputting unit 106 such that the object is held with the shape and thickness thereof being suitable for imaging. At this time, if the flat plates 1110 are parallel flat plates in pairs, the operator adjusts the interval between the flat plates 1110 while holding the parallel state. After the adjustment of the interval, the operator applies a brake to the flat plates 1110 to fix the same and prevent a change in the shape and position of the object.

In the above description, the operator controls the flat plates 1110 and fixes the holding position via the inputting unit 106 of the information processing part 1000. Alternatively, an operating unit may be provided in the photoacoustic wave signal measuring part 1100 to allow the breast to be held by technique or the like.

In step S402, the operator sets various parameters for imaging and gives instructions to start imaging via the inputting unit 106.

In step S403, the information processing part 1000 and the photoacoustic wave signal measuring unit 1100 having received the instructions from the operator execute the imaging of the object in conjunction with each other. As described in the above section of the object state monitoring unit 1005, the imaging is executed while the holding state of the object is confirmed. If there is no change in the holding state of the object, the imaging is continued. On the other hand, if there is a change in the holding state, the imaging is stopped.

In step S404, the information processing part 1000 makes an image of imaging data and displays a reconstruction image on the monitor 105.

In the above procedure, the imaging of the object is executed.

(Procedure of Information Processing)

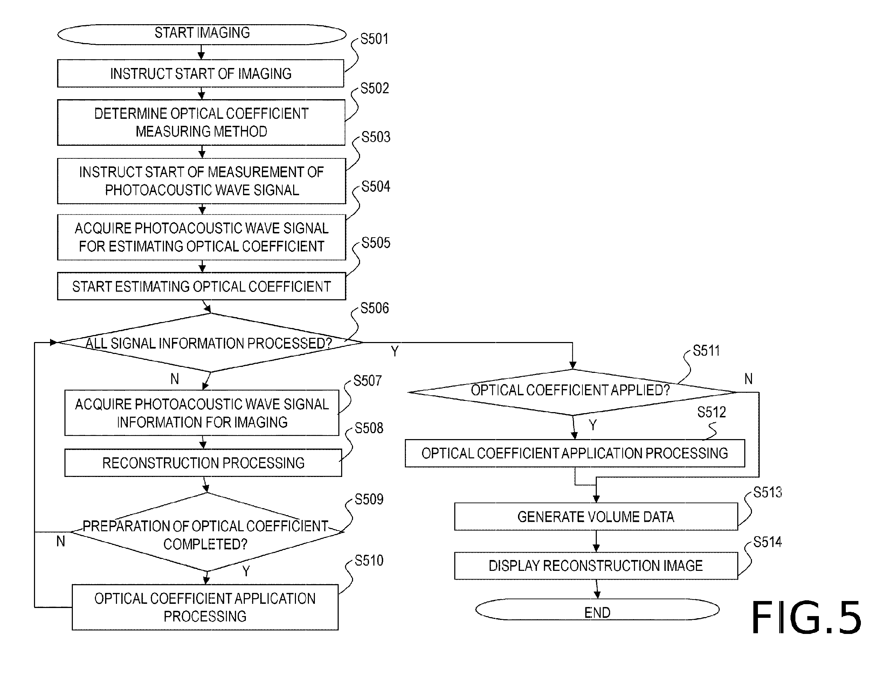

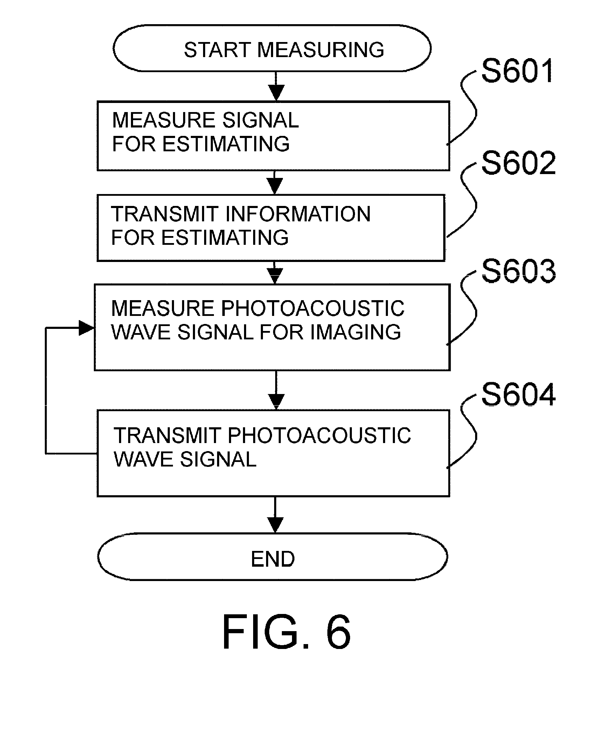

Next, a description will be given of the operations of the photoacoustic wave diagnosing apparatus according to the first embodiment of the present invention. FIG. 5 is a flowchart showing the processing procedure of the information processing part 1000 according to the first embodiment of the present invention. FIG. 6 is a flowchart showing the processing procedure of the photoacoustic wave signal measuring part 1100 according to the first embodiment of the present invention.

Using the flowcharts shown in FIGS. 5 and 6, a description will be given of the details of the imaging in step S403 of FIG. 4, i.e., the operations of each block in the imaging processing. The flowchart shown in FIG. 5 starts from a state in which the operator gives the instructions to start the imaging after having set the imaging parameters.

In step S501, the imaging instruction information acquiring unit 1001 generates imaging instruction information based on input instruction contents. The imaging instruction information may include, besides information on settings on the imaging functions of the photoacoustic wave diagnosing apparatus, information on analysis to be executed after the imaging or information on image reconstruction (reconstruction instruction information). In addition, the imaging instruction information includes information on items set in advance, besides information on settings adjusted by the operator for each time and changed for each imaging. The imaging instruction information acquiring unit 1001 transmits the generated imaging instruction information to the optical coefficient measuring method determining unit 1002.

In step S502, the optical coefficient measuring method determining unit 1002 determines, based on the imaging instruction information, whether the optical coefficient to be applied to image reconstruction is calculated from estimation based on the measurement of the photoacoustic wave signal or is calculated from the measurement. In addition, the optical coefficient measuring method determining unit 1002 determines a method for measuring or estimating the optical coefficient.

According to the embodiment, the photoacoustic wave signal is measured, and then the optical coefficient is estimated based on the measurement result. Further, in order to measure the photoacoustic wave signal, the object in the same holding state as the time of the imaging is irradiated with the light to acquire the photoacoustic wave signal for estimating the optical coefficient. According to the embodiment, a region and various settings on the measurement of the photoacoustic wave signal for estimating the optical coefficient are automatically determined based on information and settings on an imaging region specified by the imaging instruction information.

According to the embodiment, the region for acquiring the photoacoustic wave signal for estimating the optical coefficient matches the imaging region. However, the photoacoustic wave signal for estimating the optical coefficient may be acquired from a specific region different from the imaging region. In this case, the imaging instruction information is only required to include information for specifying the region for estimating the optical coefficient.

The optical coefficient measuring method determining unit 1002 generates information on the optical coefficient measuring method as optical coefficient measuring information and transmits the same to the photoacoustic wave measuring method determining unit 1003, the photoacoustic wave signal information acquiring unit 1006, and the reconstruction processing unit 1008 together with the imaging instruction information.

In step S503, the information processing part 1000 instructs the photoacoustic wave signal measuring part 1100 to start the imaging. In response to the instructions to start the imaging, each function block performs the following processing.

The photoacoustic wave measuring method determining unit 1003 determines the photoacoustic wave measuring method of the photoacoustic wave signal measuring part 1100 based on the imaging instruction information and the optical coefficient measuring information. For example, as for the control of the irradiation light, the photoacoustic wave measuring method determining unit 1003 adjusts settings on a light source, a light path, an irradiating method, or the like.

In addition, the photoacoustic wave measuring method determining unit 1003 calculates a required scanning region (receiving region) based on the imaging region and a reconstruction method specified by the operator. Further, the photoacoustic wave measuring method determining unit 1003 determines information for measuring and controlling the photoacoustic wave signal for estimating the optical coefficient (e.g., a range for measuring the photoacoustic wave signal for estimating the optical coefficient) based on the imaging instruction information and the imaging region. Furthermore, the photoacoustic wave measuring method determining unit 1003 determines the pitch of an element position on the receiving region for allowing the element of the acoustic wave detector 1005 to detect the photoacoustic wave signal required for image reconstruction.

Basically, the control of detecting the acoustic wave, correction based on acoustic characteristics inside the apparatus, and the like are performed by the photoacoustic wave signal measuring part 1100. However, acoustic wave acquiring conditions on the image quality of image reconstruction, acoustic wave acquiring conditions on the accuracy of estimating the optical coefficient, and a correction method may be determined by the photoacoustic wave measuring method determining unit 1003.

The photoacoustic wave measuring method determining unit 1003 generates photoacoustic wave measuring information including instruction information and a controlling method required to measure the photoacoustic wave signal for imaging and the photoacoustic wave signal for estimating the optical coefficient based on the information determined as described above and transmits the same to the photoacoustic wave measuring method instructing unit 1004.

Here, the embodiment describes a case in which the photoacoustic wave measuring information is generated for each imaging. Alternatively, equivalent photoacoustic wave measuring information may be generated in advance and selected. In this case, the photoacoustic wave measuring method determining unit 1003 transmits the identifier of the photoacoustic wave measuring information generated in advance to the photoacoustic wave measuring method instructing unit 1004.

Moreover, the photoacoustic wave measuring method determining unit 1003 determines the controlling method of the photoacoustic wave signal measuring part 1100 required to acquire the acoustic wave at the receiving region and generates information on the acquisition of the photoacoustic wave. The controlling method is, for example, a probe scanning method or a light irradiation controlling method. The information on the acquisition of the photoacoustic wave may include the relative positional relationship between the object 1107 held by the flat plates 1110 and the optical unit 1104 and the acoustic wave detector 1105. The information on the acquisition of the photoacoustic wave is composed of, for example, a command and a group of parameters for giving instructions to acquire the acoustic wave to the photoacoustic wave signal measuring part 1100.

Then, the photoacoustic wave measuring method instructing unit 1004 generates photoacoustic wave measuring information based on the information on the acquisition of the photoacoustic wave and transmits the same to the photoacoustic wave signal measuring part 1100 to instruct the measurement of the photoacoustic wave. However, the photoacoustic wave measuring method instructing unit 1004 inquires in advance the object state monitoring unit 1005 about the fact whether the object 1107 is in a state capable of being imaged. Then, if the object 1107 is in a state capable of being imaged, the photoacoustic wave measuring method instructing unit 1004 gives instructions to measure the photoacoustic wave to the photoacoustic wave signal measuring part 1100 and notifies the object state monitoring unit 1005 of the start of the measurement.

The object state monitoring unit 1005 monitors whether there is no change in the holding state of the object 1107 during the measurement of the photoacoustic wave by the photoacoustic wave signal measuring part 1100. The object state monitoring unit 1005 may use any monitoring unit so long as the state of the object 1107 can be monitored. For example, the object state monitoring unit 1005 may periodically communicate with the photoacoustic wave signal measuring part 1100 to inquire about the holding state of the object 1107 or may monitor the photoacoustic wave signal measuring part 1100 at all times. In monitoring the object 1107, the object state monitoring unit 1005 monitors the holding state or the like of the object 1107 fixed for the imaging.

The holding state of the object 1107 fixed for the imaging can be confirmed based on whether the measurement values of various sensors fall within prescribed thresholds. As such, there is a sensor that measures pressure on the flat plates 1110 holding the object 1107. In addition, there is a sensor that measures the distance and position of the flat plates 1110 or the like. Further, there is a sensor that measures a force indicating a braking state when the object 1107 is fixed. Furthermore, there is a sensor that detects the presence or absence of the object 1107 at each position inside the apparatus.

Moreover, the object state monitoring unit 1005 may monitor, besides the holding state of the object 1107, any change likely to exert an influence on the calculation of the optical coefficient. For example, the object state monitoring unit 1005 can monitor various measurement values and apparatus states such as temperatures inside and outside the photoacoustic wave signal measuring part 1100 and the opening states of the door and cover of the apparatus.

Here, a method for monitoring the state of the object 1107 is not limited to the installation of the object state monitoring unit 1005 as in the embodiment. For example, if a sensor measures a change in the holding state of the object 1107 during the imaging of the object 1107 by the photoacoustic wave signal measuring part 1100 or when the fixing of the object 1107 is cancelled, an error code indicating information including a change in an apparatus state that changes with the holding state and including a change in the holding state may be transmitted to the information processing part 1000.

The object state monitoring unit 1005 notifies the photoacoustic wave signal information acquiring unit 1006 of the fact as to whether there is a change in the state of the object 1107. The object state monitoring unit 1005 may perform the notification at any timing. For example, the object state monitoring unit 1005 is only required to periodically notify the photoacoustic wave signal information acquiring unit 1006 of the fact from the start to the end of the measurement of the photoacoustic wave. However, if there is a change in the state of the object 1107, the object state monitoring unit 1005 instructs the photoacoustic wave measuring method instructing unit 1004 to stop the measurement of the photoacoustic wave.

In step S504, the photoacoustic wave signal information acquiring unit 1006 receives the photoacoustic wave signal for estimating the optical coefficient from the photoacoustic wave signal measuring part 1100 and transmits the same to the optical coefficient estimating unit 1007.

In step S505, the optical coefficient estimating unit 1007 starts optical coefficient estimating processing using the photoacoustic wave signal for estimating the optical coefficient. According to the embodiment, the optical coefficient estimating unit 1007 corresponds to an optical coefficient acquiring unit.

As a method for estimating the optical coefficient, any estimating method may be applied to the embodiment of the present invention so long as the optical coefficient of the object 1107 in the same holding state as the time of the imaging is estimated in the processing.

For example, according to the estimating method described in Japanese Patent Application Laid-open No. 2011-217914, two different types of light may be transmitted to a region for estimating the optical coefficient inside the object 1107 in the same holding state as the time of the imaging to measure the photoacoustic wave of the object 1107. For example, using two types of signals for estimating the optical coefficients measured by irradiating the object 1107 with the light from two directions, the distribution of initial sound pressure is calculated for each signal. The directions include, for example, the forward direction and the backward direction as described above.

As a result, the two distribution data items of the initial sound pressure are generated based on the photoacoustic wave signals when the light is caused to reach one region for estimating the optical coefficients via the two transmission paths. Based on the fact that the ratio of the two distributions of the initial sound pressure at each position inside the region for estimating the optical coefficient becomes equal to the ratio of light amounts at the corresponding position inside the region for estimating the optical coefficient, the optical coefficients (an absorption coefficient and a scattering coefficient according to the estimating method described in Japanese Patent Application Laid-open No. 2011-217914) are approximated to each other by a Monte Carlo method or the like. The optical coefficient is estimated according to the above method, whereby the average of the absorption coefficients of the light inside the region for estimating the optical coefficient is calculated.

Note that the region for estimating the optical coefficient may not be the same in position and size as the region for measuring the photoacoustic wave for the imaging, and measuring parameters such as an integration time or the like are not necessarily the same so long as an estimated value suitable for an imaging region can be calculated.

That is, the region is only required to estimate the optical coefficient that can be applied as the average of the optical coefficients inside the imaging region.

In step S506, while all the photoacoustic wave signals for the imaging are measured, a determination is made in units of divided photoacoustic wave signal information items as to whether any unprocessed photoacoustic wave signal information exists. Until no unprocessed photoacoustic wave signal information exists, the acquisition of the photoacoustic wave signal information and image reconstruction are repeatedly performed in steps S507 to S510.

In step S507, the photoacoustic wave signal information acquiring unit 1006 acquires the photoacoustic wave signal information for the imaging from the photoacoustic wave signal measuring part 1100 and transmits the same to the reconstruction processing unit 1008. The photoacoustic wave signal information acquiring unit 1006 performs this step regardless of before and after the completion of estimating the optical coefficient.

In step S508, the reconstruction processing unit 1008 performs the image reconstruction of the photoacoustic wave signal. The image reconstruction in step S508 can be performed without the application of the optical coefficient. For example, the reconstruction processing unit 1008 calculates the distribution of the initial sound pressure of the photoacoustic wave of each voxel defining the imaging region as volume space. The distribution of the initial sound pressure is calculated based on the photoacoustic wave signal information corresponding to the region obtained by dividing the imaging region. Accordingly, the reconstruction processing unit 1008 can simultaneously perform the processing of this step even before the completion of the optical coefficient estimating processing. When the reconstruction processing unit 1008 completes the image reconstruction of the one photoacoustic wave signal information transmitted from the photoacoustic wave signal measuring part 1100, the processing proceeds to step S509.

In step S509, a determination is made as to whether the preparation of the optical coefficient has been completed, i.e., whether the optical coefficient estimating processing has been completed according to the embodiment. If the optical coefficient estimating processing has not been completed, the processing returns to step S506 to perform the processing of the next photoacoustic wave signal information. On the other hand, if the optical coefficient estimating processing has been completed, the processing proceeds to step S510.

In step S510, the reconstruction processing unit 1008 performs the image reconstruction with the application of the estimated value of the optical coefficient calculated by the optical coefficient estimating unit 1007 as the background optical coefficient. For example, the reconstruction processing unit 1008 can calculate the distribution of the light absorption coefficients at the imaging of the object 1107 from the voxel value of the distribution of the initial sound pressure calculated based on the photoacoustic wave signal information. The reconstruction processing unit 1008 calculates the distribution of the light absorption coefficients of the object 1107 by estimating the attenuation of the laser light inside the object 1107. Therefore, with the use of the accurate optical coefficient calculated based on the information measured from the object 1107 at the imaging, it is possible to generate volume data (reconstruction image) in which the value of the more accurate absorption coefficient is set as a voxel value.

If it is determined in step S506 that the image reconstruction of all the photoacoustic wave signal information items inside the imaging region has been completed, the processing proceeds to step S511.

In step S511, if the estimating processing has not been successfully performed on time in the image reconstruction and any reconstruction image data with no application of the optical coefficient exists, the processing proceeds to step S512. On the other hand, if the estimating processing has been successfully performed on time at the acquisition of the first photoacoustic wave signal information for the imaging, the processing proceeds to step S513.

In step S512, the same processing as step S510 is performed on the reconstruction image data with no application of the optical coefficient, and the processing proceeds to step S513.

In step S513, the reconstruction processing unit 1008 puts the reconstruction image data items together to generate the volume data of the entire imaging region. Here, the reconstruction image data items represent a group of voxels corresponding to each part of the imaging region reconstructed based on the photoacoustic wave signal measuring information transmitted in a divided manner. At this time, if the voxel put in the same position as each voxel inside the imaging region exists so as to extend over the plurality of reconstruction image data items, the reconstruction processing unit 1008 performs the averaging processing of each value as required. After generating the volume data, the reconstruction processing unit 1008 transmits the volume data storing the reconstruction image with the application of the optical coefficient and information on the reconstruction image to the data analyzing unit 1011. Thus, the processing proceeds to step S514.

In step S514, the data analyzing unit 1011 puts together the volume data of the reconstruction image and the information on the reconstruction image into management information and transmits the management information to the display information generating unit 1012. Using the reconstruction image data according to display settings adjusted in advance, the display information generating unit 1012 generates display image information on the reconstruction image capable of being displayed on the displaying unit 1013. Then, the display information generating unit 1012 transmits the generated display image information to the displaying unit 1013.

As an example of displaying the display image information, when the reconstruction image is displayed by multi planar reconstruction (MPR), the cross-sectional image of the reconstruction image and a boundary line showing image quality are displayed so as to overlap with each other. In addition, the display image may be displayed by volume rendering. Further, besides the display image information, other information such as text information based on the pixel value of each position of the three-dimensional reconstruction image, i.e., the voxel value of the volume data may be generated. Furthermore, the display information generating unit 1012 may generate the display image information using any display method, other analyzing functions, or the like according to instructions by the user if the display image information corresponds to the reconfiguration image. Moreover, the display image information may include texts, icons, or the like showing that the optical coefficient used for the reconstruction is obtained by estimation.

Using the transmitted display image information, the displaying unit 1013 displays the reconstruction image with the application of the optical coefficient estimated based on the state of the object 1107 at the imaging.

(Procedure of Measurement of Photoacoustic Wave Signal)

Next, using a flowchart shown in FIG. 6, a description will be given of the procedure of the measurement of the photoacoustic wave signal of the photoacoustic wave signal measuring part 1100, which is performed simultaneously with the processing of the information processing part 1000. The flowchart shown in FIG. 6 starts when the photoacoustic wave signal measuring part 1100 is instructed by the information processing part 1000 to start measuring the photoacoustic wave signal as well as the photoacoustic wave signal for estimating the optical coefficient.

In step S601, the photoacoustic wave signal measuring part 1100 measures the photoacoustic wave signal for estimating the optical coefficient. To this end, the controlling unit 1102 controls the irradiating position and irradiating timing of the light, continues the measurement of the acoustic wave in synchronization with the position of the probe, the recording timing of the detected acoustic wave, or the like, and detects the acoustic wave at each position required for an imaging region. If the controlling unit 1102 is instructed by the information processing part 1000 to stop the measurement in mid course, the controlling unit 1102 stops the measurement. Alternatively, the controlling unit 1102 may stop the measurement at its own discretion.

The photoacoustic wave signal for estimating the optical coefficient may be measured at any region so long as the region is associated with the imaging region. According to the embodiment, the region is a three-dimensional region for estimating the optical coefficient inside the imaging region specified together with the imaging region by the operator.

In the photoacoustic wave signal measuring part 1100, the controlling unit 1102 controls the position controlling unit 1106 to control the position of the optical unit 1104 and the photoacoustic wave detector 1105 and measure the photoacoustic wave signal. Then, the measurement of the photoacoustic wave signal for estimating the optical coefficient is continued until the measurement of the photoacoustic wave for the region for estimating the optical coefficient is completed. The region for estimating the optical coefficient is a three-dimensional region inside the imaging region. On the other hand, a region on the flat plates 1110 irradiated with the light from the optical unit 1104 and a region on the flat plates 1110 on which the acoustic wave detector 1105 is caused to scan the acoustic wave are two-dimensional regions on the flat plates 1110. Accordingly, the controlling unit 1102 is required to store in advance or calculate the corresponding relationship between the region for estimating the optical coefficient and the light irradiating region or the acoustic wave detecting region. After the measurement by the photoacoustic wave signal measuring part 1100, the processing proceeds to step S602.

In step S602, the controlling unit 1102 generates photoacoustic wave signal information and transmits the same to the information processing part 1000. At this time, the controlling unit 1102 also generates information for calculating a region having quantitativeness, besides the photoacoustic wave signal information. The photoacoustic wave signal information includes the photoacoustic wave signal detected at each position on the scanning surface 502 at the irradiation of the light, information on the photoacoustic wave signal, and information on the irradiation light. If the photoacoustic wave signals are detected several times at the same position, their average or central value may be used. The information on the photoacoustic wave signal includes information such as acoustic wave acquiring conditions for detecting the photoacoustic wave signal and determining the photoacoustic wave signal.

Note that when the photoacoustic wave signal for estimating the optical coefficient is transmitted to the information processing part 1000, it may be transmitted in a favorable unit or may be transmitted at a time. If the photoacoustic wave signal for estimating the optical coefficient is transmitted in a divided manner, it may be transmitted according to the type of laser irradiation (forward direction, backward direction, and two-way direction) or may be transmitted in a unit obtained by dividing the region.

In step S603, the photoacoustic wave signal measuring part 1100 measures the photoacoustic wave signal at the imaging region. The photoacoustic wave signal for imaging is measured in a favorable unit according to the size and settings of the imaging region. For example, in a case in which the acoustic wave detector 1105 is caused to move in the horizontal direction and raise its height step by step to continue the measurement, the photoacoustic wave signal measured during the movement for one step is regarded as a favorable measuring unit.

In step S604, the photoacoustic wave signal measuring part 1100 transmits the photoacoustic wave signal to the information processing part 1000. The photoacoustic wave signal measuring part 1100 repeatedly performs the processing of steps S603 and S604 and completes the same after completing the measurement of the photoacoustic wave signal required for the imaging region. In the above procedure, the optical coefficient is estimated based on the photoacoustic wave signal measured at the imaging of the object 1107.

Note that the optical coefficient measuring method determining unit 1002 and the photoacoustic wave measuring method determining unit 1003 may be included in the photoacoustic wave signal measuring part 1100. Moreover, an apparatus in which the information processing part 1000 and the photoacoustic wave signal measuring part 1100 are combined together may be used.

According to the embodiment, the reconstruction image is directly displayed on the displaying unit 1013. However, the reconstruction image may be displayed while its data is recorded via the data recording unit 1009. Further, it is also possible to temporarily store the imaging data and then display the reconstruction image later via the data acquiring unit 1010 and the data analyzing unit 1011.

<Modification>

According to the embodiment, the measurement of the photoacoustic wave signal for imaging and the measurement of the photoacoustic wave signal for estimating the optical coefficient are described as different procedures. Accordingly, the operator can acquire the optical coefficient corresponding to the state of the object with the application of any imaging setting at the imaging of the object. In addition, since the estimating processing of the optical coefficient and the measurement of the photoacoustic wave signal for imaging are simultaneously performed, the extension of total imaging processing time due to the time of the estimating processing can be eliminated. However, if the settings and the conditions on the measurement of the photoacoustic wave signal for the imaging are treated as the photoacoustic wave signal for estimating the optical coefficient, part of the same photoacoustic wave signal as the photoacoustic wave signal for the imaging is used to estimate the optical coefficient. If there is a difference between the conditions, the required measurement of the photoacoustic wave signal for estimating the optical coefficient may be performed at each section of the measurement of the photoacoustic wave signal during the measurement of the photoacoustic wave signal for the imaging.

In addition, the embodiment describes the operating procedure in which the estimating processing of the optical coefficient, the measurement of the photoacoustic wave signal for the imaging, and image reconstruction with no application of the optical coefficient are simultaneously performed in order to reduce total imaging time. However, unlike the operating procedure described in the embodiment, the measurement of the photoacoustic wave signal for estimating the optical coefficient and the estimating processing of the optical coefficient may be performed after the measurement of the photoacoustic wave signal for the imaging. Moreover, even if the respective processing steps are not simultaneously performed but the measurement of the photoacoustic wave signal, image reconstruction, and the estimating processing of the optical coefficient are individually performed one after another, the essential feature of the embodiment of the present invention is not changed.

<Second Embodiment>

According to the first embodiment, the measurement of the photoacoustic wave signal for the imaging and the measurement and estimating of the photoacoustic wave signal for estimating the optical coefficient are performed while confirming whether there is no change in the holding state of the object held at the imaging. In addition, the estimated value of the optical coefficient is applied to the image reconstruction. However, it is not necessarily required to estimate the optical coefficient based on the measurement result of the photoacoustic wave signal according to the embodiment of the present invention. According to a second embodiment, a unit that measures the optical coefficient is added to the photoacoustic wave diagnosing apparatus to measure the optical coefficient of the object while confirming whether there is no change in the state of the object held at the imaging. That is, the photoacoustic wave diagnosing apparatus according to the second embodiment measures (not estimate) the optical coefficient of the object in an imaging state and applies the same to image reconstruction.

Hereinafter, a description will be given of the operating procedure of a second embodiment with reference to the drawings. The same processing as that of the first embodiment will be simplified, but processing different from that of the first embodiment will be described.

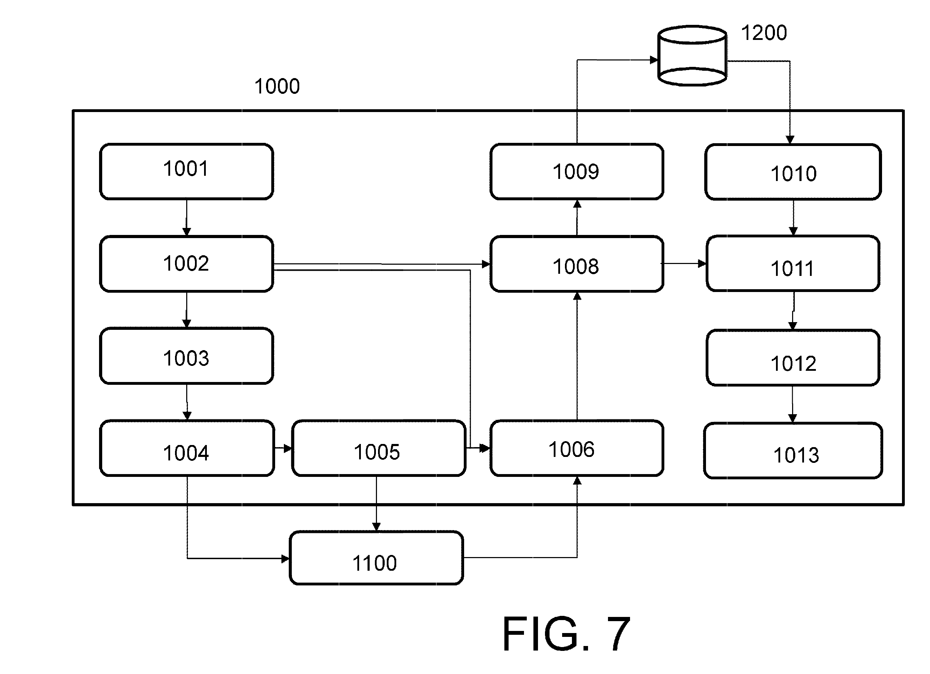

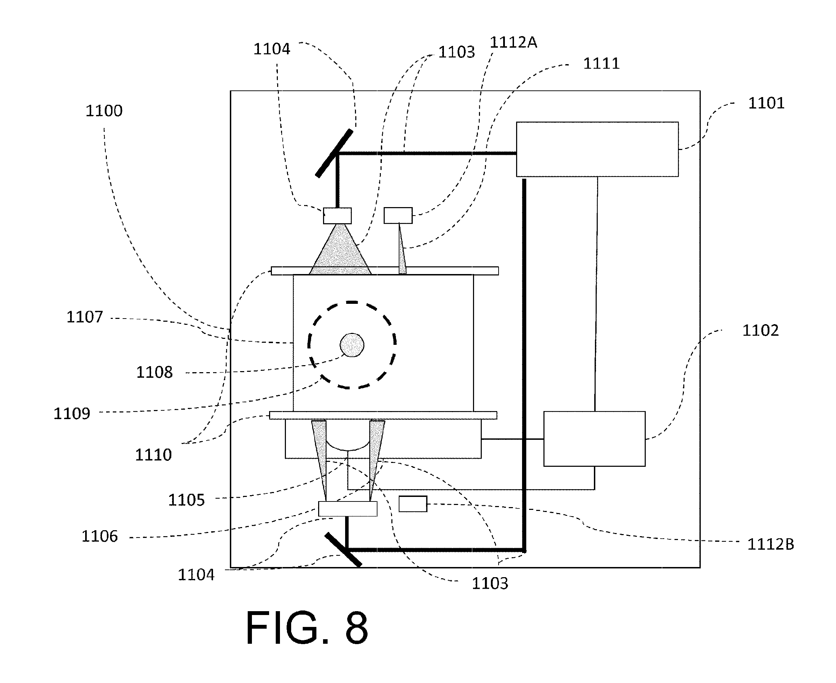

FIG. 7 is a block diagram showing the information processing part 1000 according to the second embodiment. Unlike FIG. 1, the optical coefficient estimating unit 1007 is removed from the information processing part 1000 shown in FIG. 7. FIG. 8 shows an example of the configuration of the photoacoustic wave signal measuring part 1100 according to the second embodiment. A measuring unit 1112 (including a light projector 1112A and a light receiver 1112B) that measures the optical coefficient is added to the configuration of the photoacoustic wave signal measuring part 1100 of the first embodiment shown in FIG. 3.

The optical coefficient can be measured by a general measuring unit. As an example of such a unit includes the measuring unit 1112 in which measuring light 1111 is irradiated from the light projector (indicated by 1112A in FIG. 8) such as optical fibers and transmitted light is measured by the light receiver 1112B. The measuring unit 1112 may be installed at any position. For example, the measuring unit 1112 may be movable on the flat plates 1110. That is, the measuring unit 1112 may be arranged so as to be movable on the flat plates 1110 together with the optical unit 1104 and the acoustic wave detector 1105 by the position controlling unit 1106. In this case, the measuring unit 1112 is caused to move so as to maintain a relationship in which the light receiver 1112B is arranged on the light axis of the measuring light emitted from the light projector 1112A.

Next, using the flowcharts shown in FIGS. 5 and 6, a description will be given of the operating procedure of the second embodiment focusing on the difference between the operating procedure of the first embodiment and that of the second embodiment. As in the first embodiment, the processing of the second embodiment starts from a state in which the operator gives instructions to start the imaging after having set the imaging parameters in the flowchart shown in FIG. 5.

(Procedure of Information Processing)

Since the processing of step S501 of the second embodiment is the same as that of the first embodiment, the description thereof will be omitted.

In step S502, the optical coefficient measuring method determining unit 1002 selects the method of acquiring the optical coefficient to be applied to the image reconstruction based on measurement using the measuring unit rather than estimation using the measurement result of the photoacoustic wave signal. Then, the optical coefficient measuring method determining unit 1002 adjusts various settings on a range for measuring the optical coefficient instead of settings on a region for measuring the photoacoustic wave signal for estimating the optical coefficient.

The processing of step S503 of the second embodiment is different from that of the first embodiment in that a range for measuring the optical coefficient, parameters at measurement, and the like are determined instead of a range for measuring the photoacoustic wave signal for estimating the optical coefficient. The other processing of the second embodiment is the same as that of the first embodiment.

The processing of step S504 of the second embodiment is different form that of the first embodiment in that the photoacoustic wave signal acquiring unit 1006 transmits the photoacoustic wave signal information including the measurement value of the optical coefficient of the object to the reconstruction processing unit 1008. The processing of step S505 is omitted in the second embodiment. According to the second embodiment, the photoacoustic wave signal acquiring unit 1006 corresponds to the optical coefficient acquiring unit.

Among the processing of steps S506 to S514, the processing of step S509 of the second embodiment is different from that of the first embodiment. According to the first embodiment, the preparation of the optical coefficient represents the estimating processing. On the other hand, according to the second embodiment, a determination is made as to whether the measurement value of the optical coefficient has been acquired. If the measurement value of the optical coefficient is transmitted prior to the photoacoustic wave signal information for the imaging from the photoacoustic wave signal measuring part 1000 to the information processing part 1000, the measured optical coefficient is applied at the start of the image reconstruction. On the other hand, if the measurement value of the optical coefficient is transmitted after the start of the image reconstruction, the image reconstruction with no application of the optical coefficient is preceded as in the first embodiment. Then, the optical coefficient is applied after the acquisition of the measurement value of the optical coefficient. Since the other processing of the second embodiment is same as that of the first embodiment, the description thereof will be omitted.

(Procedure of Measurement of Photoacoustic Wave Signal)

Next, focusing on the difference between the first and second embodiments, a description will be given of the procedure of the measurement of the photoacoustic wave signal of the photoacoustic wave signal measuring part 1100, which is performed simultaneously with the processing of the information processing part 1000 in the second embodiment.

Using the flowchart shown in FIG. 6, a description will be given of the processing procedure of the photoacoustic wave signal measuring part 1100 according to the second embodiment. The flowchart shown in FIG. 6 starts when the photoacoustic wave signal measuring part 1100 is instructed by the information processing part 1000 to start measuring the optical coefficient of the object and the photoacoustic wave signal.

In step S601, the photoacoustic wave signal measuring part 1100 measures the optical coefficient of the object. To this end, the controlling unit 1102 causes the measuring light 1111 to be applied from the light projector 1112A of the measuring unit 1112 to the object and calculates the optical coefficient based on the strength of the measuring light 1111 received at the light receiver 1112B.

At this time, a region for measuring the optical coefficient is set by the operator. For example, if a range for measuring the optical coefficient is set inside an imaging region, the optical coefficient is measured inside the imaging region of the object. Further, if the optical coefficient of the object outside the imaging region may be used, the optical coefficient outside the imaging region is measured. Moreover, the measurement value of the optical coefficient may be acquired for each unit region of any size inside the imaging region or may be acquired by averaging measurement values inside the region of any size. Such measurement values are only required to be suitable for the processing of the reconstruction processing unit 1008.

The processing of steps S602 to S604 of the second embodiment is different from that of the first embodiment in that photoacoustic wave signal information includes the measurement value of the optical coefficient rather than information on the photoacoustic wave signal for estimating the optical coefficient. Since the other processing of the second embodiment is the same as that of the first embodiment, the description thereof will be omitted. In the manner described above, the processing of the photoacoustic wave signal measuring part 1100 according to the second embodiment of the present invention can be performed.

Here, the second embodiment describes the procedure in which the optical coefficient is measured prior to the measurement of the photoacoustic wave signal for the imaging and the measurement value of the optical coefficient is transmitted first. However, the optical coefficient is not necessarily measured prior to the measurement of the photoacoustic wave signal. That is, the optical coefficient may be measured during or after the measurement of the photoacoustic wave signal. Moreover, the optical coefficient may be measured simultaneously with the measurement of the photoacoustic wave signal.

Further, the optical coefficient may be measured in a state in which the measuring unit 1112 is caused to move by the position controlling unit 1106 together with the acoustic wave detector 1105 and the optical unit 1104. Thus, even if the optical coefficient is measured in parallel, the second embodiment can be implemented.

In the procedure described above, the optical coefficient of the object is acquired by the measuring unit with the object remaining in the same holding state as the time of the imaging and is applied to the image reconstruction, thereby making it possible to provide an accurate reconstruction image.

As described in each of the embodiments, the photoacoustic wave diagnosing apparatus can calculate the optical coefficient of the object in the actual holding state of the object. As a result, it becomes possible to more accurately perform the calculation of sound pressure strength or the like than ever before and improve the accuracy of diagnosis.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions. Disclosed is a photoacoustic wave diagnosing apparatus including a holding unit that presses and holds an object during imaging; a photoacoustic measuring unit that measures information on the photoacoustic wave of the object pressed by the holding unit; an optical coefficient acquiring unit that acquires an optical coefficient based on the object in the pressed state; and a reconstruction unit that performs image reconstruction based on the information on a photoacoustic wave signal measured by the photoacoustic measuring unit and the optical coefficient acquired by the optical coefficient acquiring unit.

**Claims**

1. An object information acquiring apparatus, comprising:

   a holding unit that holds an object;
   an irradiating unit that irradiates the object with light;
   a photoacoustic measuring unit that measures a photoacoustic wave generated when the irradiating unit irradiates the object held by the holding unit with the light;
   an optical coefficient acquiring unit that acquires an optical coefficient of the object; and
   a processing unit that generates property information inside the object using the photoacoustic wave measured by the photoacoustic measuring unit and the optical coefficient acquired by the optical coefficient acquiring unit, wherein
   the optical coefficient acquiring unit acquires the optical coefficient by irradiating the object held by the holding unit with the light.

2. The object information acquiring apparatus according to claim 1, wherein the optical coefficient acquiring unit estimates the optical coefficient based on the photoacoustic wave that is generated when the irradiating unit irradiates the object held by the holding unit with the light and is measured by the photoacoustic measuring unit.

3. The object information acquiring apparatus according to claim 2, wherein the photoacoustic wave used for estimating the optical coefficient by the optical coefficient acquiring unit is measured prior to the measurement of the photoacoustic wave used for generating the property information inside the object.

4. The object information acquiring apparatus according to claim 2, wherein the photoacoustic wave used for estimating the optical coefficient by the optical coefficient acquiring unit is part of the photoacoustic wave measured to be used for generating the property information inside the object.

5. The object information acquiring apparatus according to claim 1, further comprising:

   a light projector that irradiates the object held by the holding unit with light; and
   a light receiver that measures the light irradiated from the light projector and passing through the object, wherein
   the optical coefficient acquiring unit calculates the optical coefficient based on the light measured by the light receiver.

6. A method for controlling an object information acquiring apparatus having a holding unit that holds an object and an irradiating unit that irradiates the object with light, the method comprising the steps of:

   measuring a photoacoustic wave generated when the irradiating unit irradiates the object held by the holding unit with the light;
   acquiring an optical coefficient of the object by irradiating the object held by the holding unit with the light; and
   generating property information inside the object using the photoacoustic wave and the optical coefficient.

FIG. 1

FIG. 2

EP 2 649 934 A1

FIG. 3

EP 2 649 934 A1

```
          ┌──────────────────┐
          │      START       │
          └──────────────────┘
                   │
                   ▼                        S401
          ┌──────────────────┐
          │  HOLD BREAST     │
          └──────────────────┘
                   │
                   ▼                        S402
          ┌──────────────────┐
          │ SET PARAMETERS   │
          │ FOR IMAGING      │
          └──────────────────┘
                   │
                   ▼                        S403
          ┌──────────────────┐
          │ IMAGING          │
          └──────────────────┘
                   │
                   ▼                        S404
          ┌──────────────────┐
          │ DISPLAY RE-      │
          │ CONSTRUCTION     │
          │ IMAGE            │
          └──────────────────┘
                   │
                   ▼
          ┌──────────────────┐
          │      END         │
          └──────────────────┘
```

# FIG. 4

FIG.5

START IMAGING — S501

INSTRUCT START OF IMAGING — S502

DETERMINE OPTICAL COEFFICIENT MEASURING METHOD — S503

INSTRUCT START OF MEASUREMENT OF PHOTOACOUSTIC WAVE SIGNAL — S504

ACQUIRE PHOTOACOUSTIC WAVE SIGNAL FOR ESTIMATING OPTICAL COEFFICIENT — S505

START ESTIMATING OPTICAL COEFFICIENT — S506

ALL SIGNAL INFORMATION PROCESSED?

N

ACQUIRE PHOTOACOUSTIC WAVE SIGNAL INFORMATION FOR IMAGING — S507

RECONSTRUCTION PROCESSING — S508

PREPARATION OF OPTICAL COEFFICIENT COMPLETED? — S509

N

Y

OPTICAL COEFFICIENT APPLICATION PROCESSING — S510

Y — S511

OPTICAL COEFFICIENT APPLIED?

N

Y

OPTICAL COEFFICIENT APPLICATION PROCESSING — S512

GENERATE VOLUME DATA — S513

DISPLAY RECONSTRUCTION IMAGE — S514

END

EP 2 649 934 A1

START MEASURING — S601

MEASURE SIGNAL
FOR ESTIMATING — S602

TRANSMIT INFORMATION
FOR ESTIMATING — S603

MEASURE PHOTOACOUSTIC
WAVE SIGNAL FOR IMAGING — S604

TRANSMIT PHOTOACOUSTIC
WAVE SIGNAL

END

FIG. 6

FIG. 7

FIG. 8

EP 2 649 934 A1

Europäisches
Patentamt

European
Patent Office

Office européen
des brevets

# EUROPEAN SEARCH REPORT

Application Number

EP 13 16 1923

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/172513 A1 (NAKAJIMA TAKAO [JP] ET AL) 14 July 2011 (2011-07-14) * figure 1 * * paragraph [0029] - paragraph [0033] * * paragraph [0049] - paragraph [0050] * | 1-6 | INV. A61B5/00 |
| X | US 2010/087733 A1 (NAKAJIMA TAKAO [JP] ET AL) 8 April 2010 (2010-04-08) * figure 6 * * paragraph [0027] - paragraph [0028] * * paragraph [0058] - paragraph [0061] * | 1-6 | |
| X | WO 2011/136153 A1 (CANON KK [JP]; MIYASATO TAKURO [JP]) 3 November 2011 (2011-11-03) * abstract * * paragraph [0028] - paragraph [0032] * | 1-4,6 | |
| X | US 2005/085725 A1 (NAGAR RON [IL] ET AL) 21 April 2005 (2005-04-21) * paragraph [0036] * * paragraph [0047] * | 1-4,6 | TECHNICAL FIELDS SEARCHED (IPC) |
| X | US 2010/094561 A1 (MASUMURA TAKAHIRO [US]) 15 April 2010 (2010-04-15) * paragraph [0014] - paragraph [0016] * * paragraph [0040] * | 1-4,6 | A61B |

The present search report has been drawn up for all claims

| Place of search | Date of completion of the search | Examiner |
|---|---|---|
| The Hague | 24 June 2013 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 16 1923

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2013

| Patent document cited in search report | | Publication date | Patent family member(s) | | Publication date |
|---|---|---|---|---|---|
| US 2011172513 | A1 | 14-07-2011 | CN<br>EP<br>JP<br>US<br>WO | 102149314 A<br>2341818 A1<br>2010088873 A<br>2011172513 A1<br>2010030043 A1 | 10-08-2011<br>13-07-2011<br>22-04-2010<br>14-07-2011<br>18-03-2010 |
| US 2010087733 | A1 | 08-04-2010 | JP<br>US | 2010088627 A<br>2010087733 A1 | 22-04-2010<br>08-04-2010 |
| WO 2011136153 | A1 | 03-11-2011 | JP<br>US<br>WO | 2011229756 A<br>2013006090 A1<br>2011136153 A1 | 17-11-2011<br>03-01-2013<br>03-11-2011 |
| US 2005085725 | A1 | 21-04-2005 | NONE | | |
| US 2010094561 | A1 | 15-04-2010 | JP<br>US | 2010088498 A<br>2010094561 A1 | 22-04-2010<br>15-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4829934 B

- JP 2011217914 A

**Non-patent literature cited in the description**

- *PHYSICAL REVIEW E,* 2005, vol. 71, 016706